# EUROPEAN PATENT SPECIFICATION

(11) **EP 2 310 060 B1**
(45) Date of publication and mention of the grant of the patent: **27.02.2013**
(21) Application number: 09772829.9
(22) Date of filing: 25.06.2009
(51) Int. Cl.: C09D 189/06, A61L 27/34, C08L 89/06

(54) **COATING METHOD FOR MEDICAL DEVICES**
BESCHICHTUNGSVERFAHREN FÜR MEDIZINISCHE VORRICHTUNGEN
PROCÉDÉ DE REVÊTEMENT POUR DISPOSITIFS MÉDICAUX

(30) Priority: 04.07.2008 EP 08159740
(43) Date of publication of application: 20.04.2011
(73) Proprietor: Fujifilm Manufacturing Europe BV, 5047 TK Tilburg (NL)
(72) Inventor: VAN DONGEN, Elisabeth, NL-5047 TK Tilburg (NL); KLUIJTMANS, Sebastianus, NL-5047 TK Tilburg (NL); BOUWSTRA, Jan, NL-5047 TK Tilburg (NL)
(86) International application number: PCT/GB2009/050735
(87) International publication number: WO 2010/001149

(56) References cited:
- EP-A- 0 166 998
- EP-A- 0 237 037
- EP-A- 1 121 947
- US-A- 5 157 111

## Description

### FIELD OF INVENTION

The present invention is in the field of medical devices for implantation. In particular this invention relates to methods for fabricating medical devices that are coated with a biocompatible material.

### BACKGROUND OF THE INVENTION

Medical devices, in particular medical devices for implantation, may be coated so that their surfaces have desired properties or effects. For example, medical devices may be coated with materials to provide beneficial surface properties. For example, medical devices are often coated with radiopaque materials to allow for fluoroscopic visualization while placed in the body. It is also useful to coat certain devices to achieve enhanced biocompatibility. Generally such coatings comprise biocompatible polymers that cause none or minimal immunogenic of inflammatory reaction within the body. Another desired property may be promotion of cell attachment. Also "soft" bioabsorbability in vivo such that the degradation of the polymer proceeds while being friendly to the surrounding tissue (e.g., less inflammatory response, and rendering lower potential for trauma upon break-up of an implant) may be considered. Examples of biocompatible polymers are polystyrenes, polyphosphoester, polyphosphazenes, aliphatic polyesters, poly 3-hydroxybutyric acid, polylactic acid, polyethylene glycol polyvinyl alcohol, polyacrylamide or polyacrylic acid, glycosaminoglycans such as hyaluronic or chitosan acid and the like, modified polysaccharides such as cellulose or starch, or polypeptides such as poly-1-lysine or advantageously extracellular matrix proteins like gelatins, collagens, elastin, or fibrin and the like or recombinant gelatin-like proteins or recombinant collagen-like proteins.

Coatings of medical devices should further have good surgical handling characteristics, have low thrombogenicity, be free from embolic complications, have no adverse affect on surrounding tissue and have features that encourage cellular ingrowth, and have suitable strength characteristics. Another desired property of a medical device coating is a low permeability to water and biological fluids. Low permeability of a medical device coating is especially desired for vessel prosthesis meaning prosthesis replacing hollow organs in humans and animals. In some cases it is also desirable that the coating contributes to such prosthetic grafts remaining pliable and preservable in a relatively dry state for extended periods prior to use.

Biocompatible, absorbable protein coatings have been developed. For example, grafts treated with collagen or gelatin are known in the art. For example US 4,784,659 describes a vessel prosthesis that is porous as such and which is sealed by impregnating it with gelatin that is cross-linked with diisocyanate. US 5,584,875 describes a method for fabricating a vascular prosthesis comprising porous textile fabric by impregnating with a gelable material such as gelatin or collagen and cross-linking the gelable material. The known methods however are highly dependent on the intrinsic gelling property of the gelatin or collagen used to achieve sufficient coating uniformity. The gelling property of natural derived gelatin or collagen can vary from batch to batch resulting in a varied coating result. Secondly methods in the prior art often make use of cross-linking agents during the coating procedure which are consequently comprised in the coating material. This causes the whole volume of coating-material to gradually gel or harden. The use of cross-linking agents in the coating material has major drawbacks. Firstly the uniformity of the coating between consecutive medical devices coated with this technique will vary as the coating material will gradually gel during the application onto the medical device. Secondly the total volume of coating material will gel within a certain time, limiting the number of medical devices that can be coated, making the method less economic.

Accordingly, a need exists for an improved method for applying a protein-based biocompatible onto a medical device. Such an improved method should result in a medical device having a highly uniform and liquid impermeable coating of proteinaceous coating material, free from dry spots and voids, and also free from areas having excess coating material. A need also exists for the resulting, substantially uniformly coated medical devices made by such a method.

EP 166998 discloses a method for coating a medical article comprising the steps of treating a substrate constituting the medical article with a solution of a compound having reactive functional groups and then treating the substrate with a water-soluble polymer to covalently bond the reactive functional group to the water-soluble polymer.

US 5,157,111 discloses a method for covalently bonding collagen to synthetic polyester fibers. To this end polyester fiber is reacted with a bi-functional cross-linking agent and the resultant polymer is then contacted with collagen.

EP 237037 discloses a method for coating a vascular prosthesis with diisocyanate cross-linked gelatin in order to seal existing pores. Preferably good gelling gelatins are used, having a Bloom strength in the range of 110 to 300.

EP 1121947 discloses a method of coating a medical article substrate comprising the steps of treating the substrate with a solution of cross-linking agent and then applying a polymer solution to the pretreated substrate.

### SUMMARY OF THE INVENTION

The present invention concerns a method for manufacturing a coated medical device. In particular, the present method relates to a method for applying a uniform coating with low water permeability comprising a proteinaceous coating material onto a medical device. Prior art methods rely on the intrinsic gelling properties of the proteinaceous coating material applied to form the initial coating which is irreversibly cross-linked afterwards or use a solution of coating material also comprising a cross-linking agent. The intrinsic gelling properties of proteins derived from natural sources can vary, leading to variable coating results. The use of cross-linking agent in the solution of coating material will initiate gelling of the whole volume of coating material used. This also adversely affects the uniformity of the coating. The present inventors surprisingly found that impregnation of a medical device with a cross-linking agent prior to the coating with a proteinaceous coating material followed by hardening of the proteinaceous material achieves high coating uniformity and low water permeability. In this fashion the method of the invention allows proteins to gel and crosslink on contact with the medical device. The method of the current invention does therefore not rely on the intrinsic gelling properties of the protein used and also allows reuse of a solution of coating material because it is not cross-linked. The present method thus provides a medical device for implantation with a uniform coating comprising proteinaceous material resulting having low water permeability.

### GENERAL DEFINITIONS

Unless defined otherwise, all technical and scientific terms used herein have the meanings as commonly understood by one of ordinary skill in the art to which the invention belongs. Although any methods and materials similar or equivalent to those described herein can be used in the practice or testing of the present invention, the preferred methods, devices, and materials are now described.

"A medical device" as is used herein means a device or product for human body reconstruction and/or an object which is implanted in the body to control drug release. This term includes absorbable devices and products.

"Water permeability" as is used herein means the passing of a volume of clean filtered liquid, with a viscosity approximating that of water, during a specified period, through a unit area of the prosthetic material under a specified pressure.

"Proteinaceous coating material" as used herein is a composition comprising a protein.

The terms "protein" or "polypeptide" or "peptide" are used interchangeably and refer to molecules consisting of a chain of amino acids, without reference to a specific mode of action, size, three-dimensional structure or origin.

"Gelatin" as used herein refers to any gelatin, whether extracted by traditional methods or recombinant or biosynthetic in origin, or to any molecule having at least one structural and/or functional characteristic of gelatin. Gelatin is currently obtained by extraction from collagen derived from animal (e.g., bovine, porcine, rodent, chicken, equine, piscine) sources, e.g., bones and tissues. The term encompasses both the composition of more than one polypeptide included in a gelatin product, as well as an individual polypeptide contributing to the gelatin material. Thus, the term recombinant gelatin as used in reference to the present invention encompasses both a recombinant gelatin material comprising gelatin polypeptides, as well as an individual gelatin polypeptide.

Polypeptides from which gelatin can be derived are polypeptides such as collagens, procollagens, and other polypeptides having at least one structural and/or functional characteristic of collagen. Such a polypeptide could include a single collagen chain, or a collagen homotrimer or heterotrimer, or any fragments, derivatives, oligomers, polymers, or subunits thereof, containing at least one collagenous domain (Gly-X-Y region). The term specifically contemplates engineered sequences not found in nature, such as altered collagen sequences, e.g. a sequence that is altered, through deletions, additions, substitutions, or other changes, from a naturally occurring collagen sequence. Such sequences may be obtained from suitable altered collagen polynucleotide constructs, etc.

Non-gelling gelatins as used herein are gelatins with Bloom strength of lower than 50g and preferably gelatins with a Bloom strength below 10g.

"'Bloom strength" as used herein is a measurement of the strength of a gel formed by a 6.67% solution (w/v) of gelatin in a constant temperature bath (10°C) over 17 hours. A standard Texture Analyzer is used to measure the weight in grams required to depress a standard 0.5 inch in diameter AOAC (Association of Official Agricultural Chemists) plunger 4 millimeters into the gel. If the weight in grams required for depression of the plunger is 200 grams, the particular gelatin has a Bloom value of 200g. (See, e.g., United States Pharmacopoeia and Official Methods of Analysis of AOAC International, 17th edition, Volume II).

A "cross-linking agent" as described herein refers to a composition comprising a cross-linker. "Cross-linker" as used herein refers to a reactive chemical compound that is able to introduce covalent intra- and extra- molecular bridges in organic molecules. The cross-linker as mentioned in the present invention may be selected from, but is not limited to aldehyde compounds such as formaldehyde and glutaraldehyde, carbodiimide, di-aldehyde di-isocyanate, epoxides, ketone compounds such as diacetyl and chloropentanedion, bis (2-chloroethylurea), 2-hydroxy-4,6-dichloro-1,3,5-triazine, reactive halogen-containing compounds disclosed in US 3,288,775, carbamoyl pyridinium compounds in which the pyridine ring carries a sulphate or an alkyl sulphate group disclosed in US 4,063,952 and US 5,529,892, divinylsulfones, and the like. S-triazine derivatives such as 2-hydroxy-4,6-dichloro-s-triazine, are well known cross-linking compounds.

Hardening as described herein is the process of further cross-linking a partly cross-linked material with the same or another cross-linking agent than used for the initial cross-linking of the material. This step can change the physical properties of the original cross-linked material to make it more durable and/or more water impermeable.

Wettability as described herein is the property of a liquid, in particular aqueous liquid, to fully envelop a solid in any shape of form, without air bubbles or leaving dry patches on the surface of the solid. In view of the invention a high wettability as described herein is that less volume of a liquid is needed to achieve a liquid film enveloping the solid at 20°C and an atmospheric pressure of about 100 kPascal compared to pure water (i.c. water containing no additives) under the same conditions. Wettability is generally higher of liquids with a density and surface tension lower than water at 20°Celsius and an atmospheric pressure of about 100 kPascal. In this way the wettability of water can be improved by the addition of surfactants.

The term "comprising" is to be interpreted as specifying the presence of the stated parts, steps or components, but does not exclude the presence of one or more additional parts, steps or components.

In addition, reference to an element by the indefinite article "a" or "an" does not exclude the possibility that more than one of the element is present, unless the context clearly requires that there be one and only one of the elements. The indefinite article "a" or "an" thus usually means "at least one".

### DETAILED DESCRIPTION OF THE INVENTION

The present invention thus concerns a method for coating a medical device, said method comprising the steps of
a. impregnating the medical device with a cross-linking agent and then
b. contacting the impregnated medical device with a proteinaceous coating material resulting in coating of the medical device with proteinaceous material and then
c. hardening the proteinaceous material coated onto the medical device by contacting with a cross-linking agent.

A particular benefit of the present method is that the initial impregnation step allows the use of lower amounts of gelatin. A further benefit of the method according to the invention is the ability to use non-gelling proteins as the coating material. Examples of such proteins are albumin, elastin, silk-fibroin, fibrin and preferably gelatin or collagen and the like. In one embodiment the proteinaceous coating material comprises non-gelling gelatin, preferably fish gelatin. In another embodiment the proteinaceous coating material comprises recombinantly produced gelatins, collagens, elastins, silk-fibroin, or recombinant gelatin-like or collagen-like proteins. Gelatin-like or collagen-like in this context means that the sequence of the protein may contain modifications leaving a sequence consisting of Gly-Xaa-Yaa (Xaa and Yaa may be any amino acid) not completely intact but without affecting the otherwise structural and functional properties of a gelatin or collagen, in particular regarding their biocompatibility. The use of recombinant gelatins is of medical benefit in comparison to the conventionally produced gelatins from animal sources. Safety issues, such as concern over potential immunogenic, e.g., antigenic and allergenic responses, have arisen. The inability to completely characterize, purify, or reproduce animal-source gelatin mixtures used currently is of ongoing concern in the pharmaceutical and medical communities. Additional safety concerns exist with respect to bacterial contamination and endotoxin loads resulting from the extraction and purification processes. Recombinantly produced gelatins are a solution to these safety concerns. Moreover the recombinant technology allows the design of gelatin-like proteins with superior characteristics for example but not limited to low immunogenicity, improved cell attachment and or controlled biodegradability. A further benefit is that the recombinantly produced gelatin-like proteins are also more uniform in structure and size which enhances the uniformity of the coating obtained using the method of the invention. EP 0926543, EP 1014176 and WO 01/34646, and also specifically the examples of EP 0926543 and EP 1014176, describe recombinant gelatins and their production methods, using methylotrophic yeasts, in particular Pichia pastoris. In one embodiment of the present invention the proteinaceous coating material comprises recombinant gelatins that are non-hydroxylated which enhance their non-gelling characteristics. The coating method of the invention is highly suitable for coating medical devices with such non-gelling recombinant gelatins whilst the methods of the prior art are not. Production of hydroxylated recombinant gelatins may be achieved by genetically engineering a host, preferably a yeast to have proline-hydroxylase activity, for example as suitably described in WO 98/18918.

In one embodiment the method of the invention comprises the impregnation of a medical device with cross-linking agent and subsequently applying the proteinaceous coating material. The impregnation of the medical device can be done by any method such as but not limited to, spraying a solution comprising cross-linking agent onto the medical device or submerging the medical device in a solution comprising cross-linking agent. Suitable cross-linking agents are those known in the art such as chemical cross-linkers selected from aldehyde compounds such as formaldehyde and glutaraldehyde, carbodiimide, di-aldehyde di-isocyanate, ketone compounds such as diacetyl and chloropentanedion, bis (2-chloroethylurea), 2-hydroxy-4,6-dichloro-1,3,5-triazine, reactive halogen-containing compounds disclosed in US 3,288,775, carbamoyl pyridinium compounds in which the pyridine ring carries a sulphate or an alkyl sulphate group disclosed in US 4,063,952 and US 5,529,892, divinylsulfones, and the like and S-triazine derivatives such as 2-hydroxy-4,6-dichloro-s-triazine. The solution of cross-linking agent may comprise a solvent other than water that allows better wettablility of the medical device and has a higher evaporation rate at room temperature. Suitable solvents are for example but not limited to: methanol, ethanol, isopropanol, acetone, tetrahydrofuran, dioxan and ethylacetate, dependent on the solubility the cross-linker comprising the cross-linking agent. The concentration of cross-linking agent is preferably at least about 1, 2, 2.5, 4.5, 5, 7.5, 10, 12, 14, 16, 18, 20, 25, 30, 35, 40, 50 weight percent. The duration of the impregnation depends on the absorption by the medical device and is preferably at least about 1, 2, 5, 8, 10, 12, 15, 20, 30, 50 to up to 60 minutes. The impregnation step can be performed at a temperature that is suitable for the cross-linking agent, above the freezing point of the chosen composition but below the boiling point of the chosen composition dependant on the solvent and cross-linker combination used. In one embodiment the impregnation is performed near atmospheric pressure but slightly higher and lower pressure can also be used preferably. In another embodiment the impregnation is preformed at low to medium vacuum for example at about 4.0 kPa (30 mmHg) or less.

In an advantageous embodiment, the impregnation step is followed by a drying step in order to evaporate the solvent of the cross-linking agent. In one embodiment the drying step is preferably performed at the same temperature as the impregnation with cross-linking material. In one embodiment the drying step is performed near atmospheric pressure but slightly higher and lower pressure can also be used. Under near atmospheric pressure conditions the relative humidity during the drying step is preferably not higher than about 80 percent. In another embodiment the drying step is preformed under reduced pressure, for example in a low to medium vacuum for example at about 4.0 kPa (30 mmHg) or less. The medical device may be dried for at least about 1, 2, 4, 8, 10, 12, 16, 18, 20 up to 24 hours.

The present method further comprises a coating step, in particular a step wherein proteinaceous coating material is applied onto the medical device, e.g. by contacting the impregnated medical device with a proteinaceous coating material resulting in coating of the medical device with proteinaceous material. This can be performed by spraying a solution of the coating material onto the medical device. In one embodiment the medical device is submerged in a solution of the proteinaceous coating material which also can be referred to as dip-coating. Suitable solvents for the proteinaceous coating material is preferably water but it may also comprise a co-solvent for example but not limited to methanol, ethanol, isopropanol, acetone, tetrahydrofuran, dioxan and ethyl acetate. The proteinaceous coating material may comprise at least 1 weight percent up to 60 weight percent of such a co-solvent. The proteinaceous coating material may comprise at least about 0.1 weight percent up to 20 weight percent of the protein of choice. More preferred is a concentration between 5 weight percent and 10 weight percent. Examples of suitable proteins, in particular biocompatible proteins, are albumin, elastin, silk-fibroin, fibrin and preferably gelatin or collagen and the like. In another preferred embodiment the biocompatible protein is a non-gelling gelatin with a Bloom strength of below about 10g. In a more preferred embodiment the proteinaceous coating material comprises a recombinant gelatin, and preferably a non-gelling recombinant gelatin with a Bloom strength of below 10g and even more preferred the proteinaceous coating material comprises a non-hydroxylated recombinant gelatin, for example such as the recombinant gelatins that are disclosed in EP 0926543, EP1014176 and WO01/34646. Preferably at least 50 weight percent of the proteinaceous coating material is non-gelling gelatin, preferably at least 70 weight percent, more preferably at least 90 weight percent of the proteinaceous coating material is non-gelling gelatin. In a further preferred embodiment functionalized recombinant gelatins for enhanced cell binding and/or with minimal immunogenicity such as for example disclosed in EP 1608681 and EP 1368056 are comprised in the proteinaceous coating material. Functionalized recombinant gelatins can be designed to have improved cell-binding properties that simulate cellular infiltration of tissues surrounding the medical device after implantation. Another characteristic of recombinant gelatins that can be tuned in advantage for the intended application is the biodegradability. In one preferred embodiment the proteinaceous coating material also comprises surfactants and/or wetting agents that lower the surface tension of the solution. This improves the wettability of the medical device being coated and uniformity of the final coating result. Examples of suitable surfactants include anionic surfactants such as alkylsulfocarboxylates, alpha -olefin sulfonates, polyoxyethylene alkyl ether acetates, N-acylaminoacids and salts thereof, N-acylmethyltaurine salts, alkylsulphates, polyoxyalkylether sulphates, polyoxyalkylether phosphates, rosin soap, castor oil sulphate, lauryl alcohol sulphate, alkylphenol phosphates, alkyl phosphates, alkyl allyl sulfonates, diethylsulfosuccinates, diethylhexylsulfosuccinates and dioctylsulfosuccinates or cationic surfactants such as 2-vinylpyridine derivatives and poly-4-vinylpyridine derivatives or amphoteric surfactants such as lauryl dimethyl aminoacetic acid betaine, 2-alkyl-N-carboxymethyl-N-hydroxyethyl imidazolinium betaine, propyldimethylaminoacetic acid betaine, polyoctyl polyaminoethyl glycine, and imidazoline derivatives. In another preferred embodiment the proteinaceous coating material also comprises a plasticizer such as but not limited to glycols, polyethylene glycols, polyols or glycerol. This is particularly advantageous for coating flexible grafts.

The acidity of the proteinaceous coating material in the method of the invention is adjusted depending on the protein and cross-link agent used. The pH of the proteinaceous coating material may range between at least about pH 4 up to about pH 10, more preferably between at least about pH 6 and up to about pH 8. The coating step can be performed at a temperature selected from but not limited to about 1, 2, 3, 4, 5, 6, 7 , 8 , 9 10, 12, 15, 17, 19, 20, 22, 25, 27, 30, 32, 35, 37, 38, 40, 45, 50, 55°C and preferably above 0°C and preferably below 60°C. It is preferred that the duration of the coating step is as short as possible. Long exposure of the proteinaceous coating material may cause the cross-linker to diffuse from the impregnated medical device into solution, possibly causing a cross-linking reaction which may adversely affect the homogeneity of the coating and cross-linking an increased volume of proteinaceous coating material over time. In one embodiment the duration of the coating step is less than 15 minutes, preferably less than 5 minutes even more preferably less than 3 minutes and even a duration of 1 minute or less may be sufficient to achieve suitable coating. In one embodiment the coating step is performed near atmospheric pressure but slightly higher and lower pressure can also be used preferably. In a preferred embodiment the dip-coating procedure is performed at a low to medium vacuum of about 4.0 kPa (30 mmHg) or less. Advantageously this may remove air bubbles that may have been formed at the surface of the medical device being coated.

In an advantageous embodiment, the coating step is followed by a drying step. In one embodiment the drying step is preferably performed at the same temperature as the coating step. Preferably the relative humidity during the drying step is not higher than about 80 percent. In one embodiment the drying step is performed near atmospheric pressure but slightly higher and lower pressure can also be used. Under near atmospheric conditions the relative humidity during the drying step is preferably not higher than about 80 percent. In another embodiment the drying step is preformed under reduced pressure, for example in a low to medium vacuum for example at about 4.0 kPa (30 mmHg) or less. The medical device may be dried for at least about 1, 2, 4, 8, 10, 12, 16, 18, 20 up to 24 hours.

The method according to the invention further comprises a hardening step, in particular the step of hardening the proteinaceous material coated onto the medical device. This hardening step comprises contacting the biocompatible protein coated medical device with a cross-linking agent in order to further harden the coating. Advantageously such a hardening step enhances the durability of the coated medical device. Also the hardening step advantageously decreases water permeability of the coating of the medical device and preferably renders the coating "watertight", e.g. impermeable for bodily fluids. The contacting with cross-linking agent can be carried out by any suitable method such as but not limited to, spraying a solution comprising cross-linking agent onto the medical device or submerging the medical device in a solution comprising cross-linking agent. Suitable cross-linking agents can be selected but are not limited to the cross-linking agents already described herein. Suitable cross-linking agents are those known in the art such as chemical cross-linkers selected from aldehyde compounds such as formaldehyde and glutaraldehyde, carbodiimide, di-aldehyde di-isocyanate, epoxides, ketone compounds such as diacetyl and chloropentanedion, bis (2-chloroethylurea), 2-hydroxy-4,6-dichloro-1,3,5-triazine, reactive halogen-containing compounds disclosed in US 3,288,775, carbamoyl pyridinium compounds in which the pyridine ring carries a sulphate or an alkyl sulphate group disclosed in US 4,063,952 and US 5,529,892, divinylsulfones, and the like and S-triazine derivatives such as 2-hydroxy-4,6-dichloro-s-triazine. The solution of cross-linking agent may comprise a solvent other than water that allows better wettablility of the medical device and has a higher evaporation rate at room temperature. Suitable solvents are for example but not limited to methanol, ethanol, isopropanol, acetone, tetrahydrofuran, dioxan and ethyl acetate. The concentration of cross-linking agent is preferably at least about 0.01, 0.03, 1, 3, 5 or 10 weight percent. In another preferred embodiment the cross-linking agent also comprises a plasticizer such as, but not limited to glycols, polyethylene glycols, polyols or glycerol. The duration of the hardening step according to the invention is preferably at least about 5, 6, 7, 8, 9, 10, 15 hours. The hardening step can be performed at a temperature selected from but not limited to about 1, 2, 3, 4, 5, 6, 7 , 8 , 9 10, 12, 15, 17, 19, 20, 22, 25, 27, 30, 32, 35, 37, 38, 40, 45, 50, 55°C and preferably above 0°C and preferably below 60°C. In one embodiment the hardening step is performed near atmospheric pressure but slightly higher and lower pressure can also be used. In another embodiment the hardening step is performed at near vacuum for example at about 4.0 kPa (30 mmHg) or less.

In an advantageous embodiment, the hardening step is followed by a drying step in order to evaporate the solvent of the cross-linking agent. In one embodiment the drying step is preferably performed at the same temperature as the hardening step. In one embodiment the drying step is performed near atmospheric pressure but slightly higher and lower pressure can also be used. Under near atmospheric conditions the relative humidity during the drying step is preferably not higher than about 80 percent. In another embodiment the drying step is preformed under reduced pressure, for example in a low to medium vacuum for example at about 4.0 kPa (30 mmHg) or less. The medical device may be dried for at least about 1, 2, 4, 8, 10, 12, 16, 18, 20 up to 24 hours.

In another embodiment an additional coating step is performed in-between the initial coating step b. as described above and the hardening step c. as described above. Preferably the additional coating step is carried out after a drying step after the first coating step b. The additional coating step can be performed essentially similar to the first coating step, and advantageously is followed by a drying step as describe above. Advantageously ingredients or parameters can be varied in the additional coating step, in particular it is beneficial to adjust the acidity on the proteinaceous coating material. The acidity of the proteinaceous coating material in the additional coating step is preferably adjusted to the optimal pH for the cross-linking agent used in the subsequent hardening step. In one embodiment the pH of the proteinaceous coating material in the additional coating step is between a 8 and 11, preferably the pH is about 10.

In one embodiment the cross-linking agent in the initial impregnation step of the medical device and the cross-linking agent of the hardening step are the same cross-linking agent, preferably selected from suitable cross-linking agents described herein. In a preferred embodiment the cross-linking agent is a carbodiimide or a diisocyanate. In another embodiment the cross-linking agent of the initial impregnation step of the medical device and the cross-linking agent of the hardening step are different cross-linking agents, preferably selected from suitable cross-linking agents described herein. In a more preferred embodiment the different cross-linking agents for the impregnation step and the hardening step are selected from a carbodiimide and a diisocyanate. Preferably the cross-linking agent in the impregnation step is a carbodiimide and a diisocyanate, preferably a carbodiimide, and the cross-linking agent in the hardening step is a diisocynate. More preferably the impregnation step is performed using a water soluble carbodiimide, preferably 1-ethyl-3-(3-dimethylaminopropyl)carbodiimide hydrochloride (EDC) and the hardening step is performed using a diisocyanate, preferably 1,6-hexamethylene diisocyanate.

The method of the invention can be used for coating any suitable type of medical device such as but not limited to e.g., absorbable sutures, absorbable clips, absorbable staples, absorbable pins, absorbable rods (for repairing broken bones), slow absorbable beads (for use as dermal filler), absorbable joints, absorbable sponges, hemostats, absorbable adhesives and absorbable drug control/release devices. Non-absorbable medical devices and products can also suitably be coated with the present method, especially medical devices designed for bone-repair e.g., acetabular or tibia components of joint prostheses. Also suitable to be coated according to the present method are dental implants. The method of the invention is especially suitable for coating medical devices comprising woven or nonwoven materials such as absorbable fabrics or meshes (e.g. for hernia repair) and vessel prostheses and in particular absorbable vascular grafts. The low permeability of a vessel prosthesis coating to water and biological fluids is a highly desired property and is readily achievable with the method of the invention. Also medical devices such as needles and catheters can suitable be coated according to the method of the present invention, for example to preferably provide an anti-microbial coating.

The invention will be explained in more detail in the following, non-limiting examples.

### EXAMPLES

Medical devices coated with biocompatible protein were prepared as follows.

### 1) Impregnating vascular graft with EDC

1-Ethyl-3-[3-dimethylaminopropyl]carbodiimide hydrochloride (EDC) was dissolved in ethanol. Vascular grafts of velour woven fine polyester fibers with a length of 25 mm were impregnated with:
25 wt% EDC (= 1 gram EDC in 3 gram EtOH);
2.5 wt% EDC;
0.25 wt% EDC.

Impregnation was carried out at room temperature under vacuum conditions and duration varied from 5-10 minutes. Higher concentrations of cross-linking agent eventually led to better coating results as determined by the water permeability of the vascular graft.

Similar results were obtained with EDC dissolved in water. Due to the slow evaporation of water and the better wettability of the grafts with ethanol, the latter was preferred.

### 2) Drying of the impregnated grafts

After impregnation, grafts were dried at room temperature under vacuum until ethanol was fully evaporated

### 3) Dip-coating

The impregnated vascular grafts were coated with 7.5 wt% recombinant gelatin P4 (Werten et al. (2001, Protein Engineering 14:447-454) or the RGD enriched sequence disclosed in EP 1608681 dissolved in 15 wt% glycerol in water. The pH of the solution was adjusted with HCl or NaOH. The pH dependence of the cross-linking procedure was investigated and values for the pH of 4, 6, 8 and 10 were tested. A low pH (pH 4-6) is best for EDC cross-linking, but a high pH is preferable for 1,6-hexamethylene diisocyanate (HMDIC) crosslinking (pH 10). Best results were obtained with a pH of 6 or 8. Grafts were coated short under vacuum conditions (3.6 kPa (27 mmHg)) at 40°C. The time of coating was less than 5 minutes; when the graft was totally wetted and all air bubbles were removed, the graft was taken out of the coating solution and dried. In case air bubbles were persistent, vacuum conditions were applied.

### 4) Drying

The grafts were dried overnight at room temperature.

At this point the vascular grafts were analyzed for uniformity using scanning electron microscopy and for the water permeability using the method described at point 9.

The grafts were found to be adequately coated with gelatin however it was found that the grafts were to some extent permeable to water, rendering these not immediately suitable as vascular prostheses.

### 5) Additional dip-coating step

In order to make the grafts less permeable to water, to several grafts a second layer of recombinant gelatin was applied. Dip-coating as described above under 3) was carried out at pH 10 and lower concentrations of recombinant gelatin were used, in particular concentrations of 2 and 5 wt% dissolved in 15 wt% glycerol in water. The higher pH of 10 was used to be of advantage in the subsequent hardening step for HMDIC cross-linking.

### 6) Drying

The grafts from step 5) were also dried overnight at room temperature.

### 7) Hardening step

The grafts obtained from step 4) and step 6) were submerged in 15 ml of 1 wt% HMDIC in 15% glycerol in isopropanol (IPA). Cross-linking was carried out at room temperature and duration was varied from 6 to 9 hours.

As a control no cross-linker was added to 15% glycerol in IPA.

### 8) Drying

The grafts were dried overnight at room temperature.

Again the hardened grafts were analyzed for uniformity using scanning electron microscopy and for the water permeability using the method described at step 9 and it was found that the hardening step for the grafts obtained after step 4) as well as for the grafts obtained from step 6) resulted in essentially water-impermeable coatings. Including step 5) and also using higher concentrations of recombinant gelatin in step 5) increases stiffness of the vascular grafts.

### 9) Determination of water permeability

A set of adapters specific for the internal diameter of the graft to be tested were used to mount the coated grafts. This graft adapter assembly was connected to a fixture which allows one end of the graft to extend freely while pressurized. The fixture was connected to a pressure-regulated system capable of delivering water at a pressure greater than 16 kPa (120 mmHg). The graft was pressurized to 16 kPa. The flow was allowed to stabilize and the occurrence of leakage through the graft wall was determined for 60 seconds.

### RESULTS

**Table 1: Water permeability of grafts impregnated with EDC without hardening (step 4)**

| impregnation with EDC (step 1) | pH of 7.5% RGD enriched peptide when DIP coated (step 3) | | | |
|---|---|---|---|---|
| | pH 4 | pH 5 | pH 6 | pH 7 |
| no EDC | -- | -- | -- | -- |
| 0.25% EDC | - | - | - | - |
| 2.5% EDC | +/- | + | + | +/- |
| 25% EDC | +/- | + | + | + |

**Table 2: Water permeability of grafts with EDC impregnation and hardening with 1% HMDIC (step 8)**

| impregnation with EDC (step 1) | pH of 7.5% RGD enriched peptide DIP coated (step 3) | | | |
|---|---|---|---|---|
| | pH 4 | pH 6 | pH 8 | pH 10 |
| no EDC | - | - | - | - |
| 0.25% EDC | - | - | - | - |
| 2.5% EDC | +/- | ++ | ++ | +/- |
| 25% EDC | +/- | ++ | ++ | + |

## Claims

1. A method for coating of a medical device comprising the steps of
a. impregnating the medical device with a cross-linking agent and then
b. contacting the impregnated medical device with a proteinaceous coating material resulting in coating of the medical device with a partially cross-linked proteinaceous material and then
c. hardening the proteinaceous material coated onto the medical device by contacting with a further different cross-linking agent to that used in step a; and
wherein the acidity of the proteinaceous coating material is adjusted depending on the protein and cross-linking agent used.

2. The method according to claim 1 wherein the cross-linking agent comprises a carbodiimide.

3. The method according to claim 1 or 2 wherein the cross-linking agent comprises a diisocyanate.

4. The method according to any one of claims 1-3, wherein the cross-linking agent of step a. comprises a diisocyanate and the cross-linking agent of step c. comprises a carbodiimide.

5. The method according to any one of claims 1-4 wherein the cross-linking agent of step a. comprises a carbodiimide and the cross-linking agent of step c. comprises a diisocyanate.

6. The method according to any one of claims 1-5 wherein the proteinaceous coating material of step b comprises collagen and/or gelatin.

7. The method according to any one of claims 1-7 wherein the proteinaceous coating material of step b comprises non-gelling gelatin.

8. The method according to claim 7 wherein the non-gelling gelatin comprises a recombinant gelatin-like protein.

9. The method according claim 7 or 8 wherein the non-gelling gelatin has a Bloom strength of lower than 50g.

10. A medical device for implantation comprising a biocompatible coating obtained by the method according to any one of claims 1-9.

11. The medical device according claim 10, wherein the medical device is a vascular prosthesis.

12. The medical device according claim 10, wherein the medical device is a bone repair graft.

13. The medical device according claim 10, wherein the medical device is a dental implant.

14. The medical device according claim 10, wherein the medical device is a catheter.

## Patentansprüche

1. Verfahren zum Beschichten einer medizinischen Einheit, umfassend die Schritte
a. Imprägnieren der medizinischen Einheit mit einem Vernetzungsmittel und dann
b. in Kontakt bringen der imprägnierten medizinischen Einheit mit einem proteinösen Beschichtungsmaterial, um Beschichten der medizinischen Einheit mit einem teilvernetzten proteinösen Material zu ergeben, und dann
c. Härten des auf die medizinische Einheit geschichteten proteinösen Materials durch in Kontakt bringen mit einem weiteren Vernetzungsmittel, das von dem in Schritt a verwendeten verschieden ist; und
wobei die Acidität des proteinösen Beschichtungsmaterials abhängig von dem verwendeten Protein und Vernetzungsmittel eingestellt wird.

2. Verfahren gemäß Anspruch 1, wobei das Vernetzungsmittel ein Carbodiimid umfasst.

3. Verfahren gemäß Anspruch 1 oder 2, wobei das Vernetzungsmittel ein Diisocyanat umfasst.

4. Verfahren gemäß einem der Ansprüche 1-3, wobei das Vernetzungsmittel von Schritt a ein Diisocyanat umfasst und das Vernetzungsmittel von Schritt c ein Carbodiimid umfasst.

5. Verfahren gemäß einem der Ansprüche 1-4, wobei das Vernetzungsmittel von Schritt a ein Carbodiimid umfasst und das Vernetzungsmittel von Schritt c ein Diisocyanat umfasst.

6. Verfahren gemäß einem der Ansprüche 1-5, wobei das proteinöse Beschichtungsmaterial von Schritt b Collagen und/oder Gelatine umfasst.

7. Verfahren gemäß einem der Ansprüche 1-6, wobei das proteinöse Beschichtungsmaterial von Schritt b nichtgelierende Gelatine umfasst.

8. Verfahren gemäß Anspruch 7, wobei die nichtgelierende Gelatine ein rekombinantes Gelatine-artiges Protein umfasst.

9. Verfahren gemäß Anspruch 7 oder 8, wobei die nichtgelierende Gelatine eine Bloom-Festigkeit von unter 50 g aufweist.

10. Medizinische Einheit für die Implantation, umfassend eine biokompatible Beschichtung, erhalten durch das Verfahren gemäß einem der Ansprüche 1-9.

11. Medizinische Einheit gemäß Anspruch 10, wobei die medizinische Einheit eine Gefäßprothese ist.

12. Medizinische Einheit gemäß Anspruch 10, wobei die medizinische Einheit ein Knochenreparatur-Transplantat ist.

13. Medizinische Einheit gemäß Anspruch 10, wobei die medizinische Einheit ein Dentalimplantat ist.

14. Medizinische Einheit gemäß Anspruch 10, wobei die medizinische Einheit ein Katheter ist.

## Revendications

1. Procédé de revêtement d'un dispositif médical comprenant les étapes consistant à :
a. imprégner le dispositif médical d'un agent de réticulation puis
b. mettre en contact le dispositif médical imprégné avec un matériel de revêtement protéique, conduisant à un enrobage du dispositif médical avec un matériel protéique partiellement réticulé puis
c. durcir le matériel protéique enduit sur le dispositif médical en le mettant en contact avec un agent de réticulation supplémentaire différent de celui utilisé à l'étape a ; et
dans lequel l'acidité du matériel de revêtement protéique est ajustée en fonction de la protéine et l'agent de réticulation utilisés.

2. Procédé selon la revendication 1, dans lequel l'agent de réticulation comprend un carbodiimide.

3. Procédé selon la revendication 1 ou la 2, dans lequel l'agent de réticulation comprend un diisocyanate.

4. Procédé selon l'une quelconque des revendications 1 à 3, dans lequel l'agent de réticulation de l'étape a. comprend un diisocyanate et l'agent de réticulation de l'étape c. comprend un carbodiimide.

5. Procédé selon l'une quelconque des revendications 1 à 4, dans lequel l'agent de réticulation de l'étape a. comprend un carbodiimide et l'agent de réticulation de l'étape c. comprend un diisocyanate.

6. Procédé selon l'une quelconque des revendications 1 à 5, dans lequel le matériel de revêtement protéique de l'étape b comprend du collagène et/ou de la gélatine.

7. Procédé selon l'une quelconque des revendications 1 à 6, dans lequel le matériel de revêtement protéique de l'étape b comprend une gélatine non gélifiante.

8. Procédé selon la revendication 7, dans lequel la gélatine non gélifiante comprend une protéine du type gélatine recombinante.

9. Procédé selon la revendication 7 ou la 8, dans lequel la gélatine non gélifiante a un degré Bloom inférieur à 50 g.

10. Dispositif médical destiné à être implanté, comprenant un revêtement biocompatible obtenu par le procédé selon l'une quelconque des revendications 1 à 9.

11. Dispositif médical selon la revendication 10, dans laquelle le dispositif médical est une prothèse vasculaire.

12. Dispositif médical selon la revendication 10, dans laquelle le dispositif médical est un greffon pour réparation osseuse.

13. Dispositif médical selon la revendication 10, dans laquelle le dispositif médical est un implant dentaire.

14. Dispositif médical selon la revendication 10, dans laquelle le dispositif médical est un cathéter.
